# EUROPEAN PATENT APPLICATION

(11) **EP 4 591 852 A1**
(43) Date of publication of application: **30.07.2025**
(21) Application number: 24214106.7
(22) Date of filing: 20.11.2024
(51) Int. Cl.: A61K 8/92, A61K 8/9789, A61Q 5/00, A61Q 7/00, A61K 36/28, A61K 36/53, A61K 36/82, A61K 36/8962, A61P 17/00, A61P 17/14, A61K 47/44, A61K 9/00, A61K 8/9794

(54) **A FORMULATION FOR TREATING AND PREVENTING HAIR LOSS**

(30) Priority: 21.11.2023 IL 30873723
(71) Applicant: Dr. Barr - Medicine As Art LTD, 6901645 Tel Aviv (IL)
(72) Inventor: BARR, Ehud, 6423811 Tel Aviv (IL)
(74) Representative: Modiano, Gabriella Diana

(57) **Abstract**

The invention concerns herbal hair care formulations, as well as their use for preventing, attenuating, or treating hair loss, and improving hair health and appearance.

## Description

### TECHNOLOGICAL FIELD

The present disclosure generally relates to hair care products. More particularly, the invention relates to hair oil for promoting hair health and growth.

### BACKGROUND ART

References considered to be relevant as background to the presently disclosed subject matter are listed below:
1. Natarelli et al., J. Clin. Med 12(3) : 893 (2023)

Acknowledgement of the above references herein is not to be inferred as meaning that these are in any way relevant to the patentability of the presently disclosed subject matter.

### BACKGROUND

The life cycle of a single hair lasts about two years and is composed of three primary phases: anagen, telogen, and catagen. Anagen, the hair growth phase, is a highly mitotic phase characterized by the production of a hair shaft from the hair follicle. At the anagen phase the hair is strong and not prone to shedding, whereas catagen and telogen describe regression and the resting phase of the follicle, respectively, ultimately resulting in hair shedding. A variety of factors promote anagen to telogen transition, including inflammation, hormones, stress, nutritional deficiency, poor sleep quality, and cellular division inhibiting medication (Natarelli et al. 2023).

Andro genetic type hair loss is a widespread phenomenon which affects young men and with age progression appears in most men.

Women are also affected by Andro genetic hair loss, which may commence at the beginning of menopause. It may also appear in women at a younger age due to hormonal imbalance, for example as a side effect of certain medicaments, or because of various disorders such as polycystic ovary syndrome.

There are several oral medicaments for treating hair loss or baldness. For example, Propecia, a prescription tablet taken once a day by men with male pattern baldness. It reduces the effect of hormones on hair follicles, which can prevent and even reverse hair loss. It is effective in 90% of men over five years, and its effects are often seen after three-six months. However, the use of Propecia may be associated with side effects such as breast tenderness or swelling, decreased libido, dizziness, ejaculatory disorder, erectile dysfunction, headache, male breast cancer and skin rash or hives.

5-alpha-reductase inhibitors (e.g., finasteride and dutasteride) are also used for treating androgenic alopecia. These compounds act by locally inhibiting dihydrotestosterone (DHT), however their effect becomes apparent only after several months of treatment.

Rogaine ^{®} is a topically administered scalp foam for the treatment of hereditary hair loss however it is associated with side effects such as skin irritations and irreversible growth of unwanted facial hair, and failure to comply with the treatment due to complicated use instructions as the topical solution has to be applied twice a day on dry hair and makes the hair dump and sticky and affects the longevity of a hairdo and the visibility of clean hair and sculp.

### GENERAL DESCRIPTION

In a first aspect, the present invention provides a hair care formulation comprising:
(**a**) base oil;
(**b**) Arnica montana extract;
(**c**) green tea extract;
(**d**) Salvia rosmarinus extract; and
(**e**) onion oil.

In one embodiment, said base oil comprises almond oil.

In one embodiment, the Arnica montana extract is present in an amount of between about 45% and about 55%, or at about 50%, volume/volume (v/v) of the formulation.

In one embodiment, said green tea extract is present in an amount of between about 10% and about 20%, or about 15%, volume/volume (v/v) of the formulation.

In one embodiment, said Salvia rosmarinus extract is present in an amount of between about 10% and about 20%, or at about 15%, volume/volume (v/v) of the formulation.

In one embodiment, said onion oil is present in an amount of between about 2.5% and about 7.5%, or at about 5%, volume/volume (v/v) of the formulation.

In one embodiment, said formulation is administered topically.

In one specific embodiment, the hair care formulation of the invention comprises:
(**a**) almond oil;
(**b**) 50% v/v Arnica montana extract;
(**c**) 15% v/v green tea extract;
(**d**) 15% v/v Salvia rosmarinus extract; and
(**e**) 5% v/v onion oil.

In one embodiment, the hair care formulation of the invention is for use in preventing, attenuating, or treating hair loss in a human subject.

In one embodiment, said human subject suffers from a condition selected from a group consisting of androgenic balding, telogen effluvium, medication-induced hair loss, scalp inflammation, and ichthyosis vulgaris.

In one embodiment, the hair care formulation of the invention is for use in improving hair health and appearance in a human subject.

In one embodiment, the hair care formulation of the invention is for use in reducing scalp oiliness and/or sensitivity.

In another aspect, the present invention provides a kit comprising:
(**a**) the hair care formulation of the invention; and
(**b**) instructions for use in preventing, attenuating, or treating hair loss, for use in improving hair health and appearance, or for use in reducing scalp oiliness and/or sensitivity.

In one embodiment, said instructions comprise massaging the formulation onto the scalp and keeping the formulation on the scalp for between about 40 minutes and several hours (e.g., overnight), prior to removal by washing the hair.

### BRIEF DESCRIPTION OF THE DRAWINGS

For better understanding the subject matter that is disclosed herein and to exemplify how it may be carried out in practice, embodiments will now be described, by way of non-limiting example only, with reference to the accompanying drawings, in which:

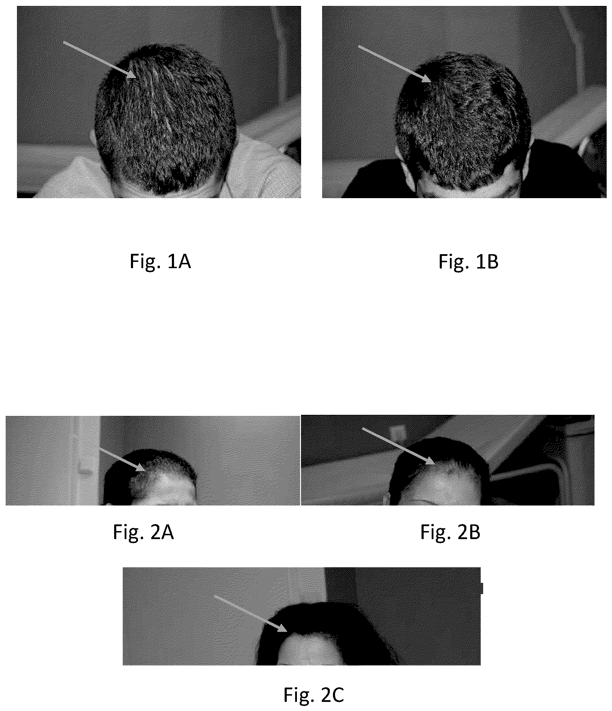

**Figure 1** is a photograph of the scalp of a male subject taken before treatment (Fig. 1A) and after 6 months of treatment (Fig. 1B) with an exemplary formulation of the present invention. Arrows point to the crown area of the scalp in which a significant difference can be observed in the amount of hair growth.
**Figure 2** is a photograph of the scalp of a female subject taken before treatment (Fig. 2A), after 3.5 months of treatment (Fig. 2B) and after 8 months of treatment (Fig. 2C) with an exemplary formulation of the present invention. Arrows point to the hair line area of the scalp in which a significant difference can be observed in the amount of hair growth.

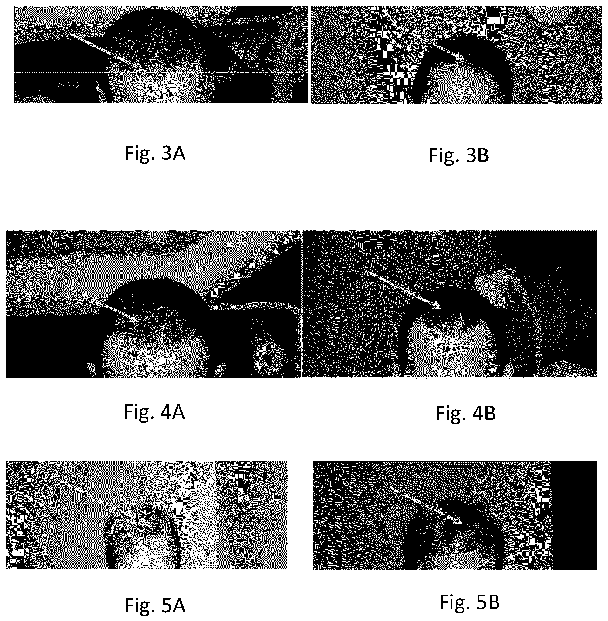

**Figure 3** is a photograph of the scalp of a male subject taken before treatment (Fig. 3A) and after 6 months of treatment (Fig. 3B) with an exemplary formulation of the present invention. Arrows point to the hair line area of the scalp in which a significant difference can be observed in the amount of hair growth.
**Figure 4** is a photograph of the scalp of a male subject taken before treatment (Fig. 4A) and after 6 months of treatment (Fig. 4B) with an exemplary formulation of the present invention. Arrows point to the hair line area of the scalp in which a significant difference can be observed in the amount of hair growth.
**Figure 5** is a photograph of the scalp of a male subject taken before treatment (Fig. 5A) and after 6 months of treatment (Fig. 5B) with an exemplary formulation of the present invention. Arrows point to the hair line area of the scalp in which a significant difference can be observed in the amount of hair growth.

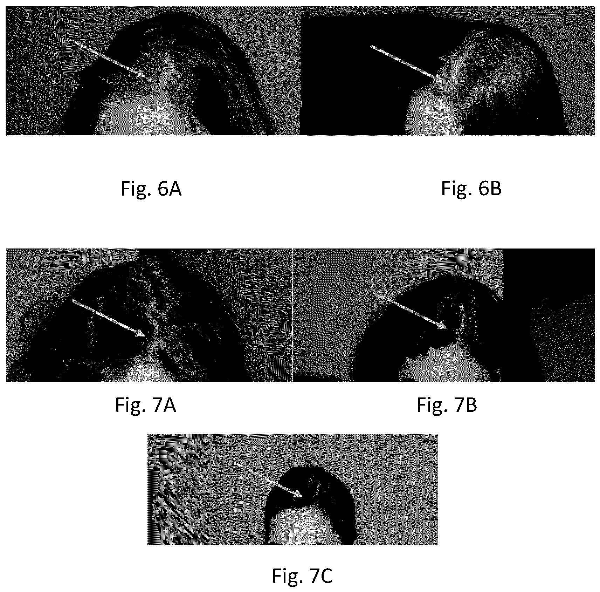

**Figure 6** is a photograph of the scalp of a female subject taken before treatment (Fig. 6A) and after 7 months of treatment (Fig. 6B) with an exemplary formulation of the present invention. Arrows point to the hair line area of the scalp in which a significant difference can be observed in the amount of hair growth.
**Figure 7** is a photograph of the scalp of a female subject taken before treatment (Fig. 7A), after 2.5 months of treatment (Fig. 7B) and after 6 months of treatment (Fig. 7C) with an exemplary formulation of the present invention. Arrows point to the hair line area of the scalp in which a significant difference can be observed in the amount of hair growth.

### DETAILED DESCRIPTION OF EMBODIMENTS

The present invention is based on the surprising finding that a unique formulation comprising a combination of herbal extracts and oil prevents hair loss and induces renewed hair growth when applied topically onto the scalp of subjects suffering from hair loss resulting from various etiologies.

The formulation was found to be effective in inhibiting hair shedding and inducing hair growth in subjects with various underlying conditions including androgenic balding, telogen effluvium, medication-induced hair loss, scalp inflammation, and ichthyosis vulgaris, regardless of their age group. In addition, the formulation improves the general health and appearance of the hair, providing moisture which is beneficial to the keratin structure and inhibits environment-induced oxidation, and oxidation which results from various cosmetic procedures. Thus, administration of the formulation results in a better quality of the hair, namely in a healthier, brighter, and thick appearance of the hair.

As used herein the term ***"androgenic balding"*** refers to androgenic alopecia or male pattern baldness, a genetically predetermined disorder in which the hair follicles around the crown are destroyed due to an excessive response to androgens (e.g., dihydrotestosterone). This process affects Caucasian males at different levels of severity, women after menopause, as well as young women suffering from hormonal imbalance.

As used herein the term ***"telogen effluvium"*** refers to excessive shedding of resting or telogen hair after metabolic stress, hormonal changes, or medication, including for example, in women after childbirth.

As used herein the term ***"medication-induced hair loss"*** refers to hair loss that occurs as a side effect of various drugs, such drugs may cause anagen shortening. These drugs include but are not limited to psychopharmaceuticals (e.g., anti-depressants, such as selective serotonin reuptake inhibitors (SSRI) or mood stabilizers such as lithium, valproic acid or divalproex, and carbamazepine), high blood pressure medications, such as beta blockers, ACE inhibitors, and diuretics.

As used herein the term ***"hair health and appearance"*** refers to increasing the brightness and thickness of the hair, as well as increasing the hair growth rate, improving the scalp condition and keratin structure.

The formulation of the invention was also shown to balance oil production in the scalp and thereby to reduce oiliness. Oiliness of the scalp is caused by accumulation of sebum. Sebum is an oily, waxy substance produced by sebaceous glands. It is necessary for a healthy scalp, but when produced in excess the oil builds up and causes greasy hair and dandruff. Accordingly, the formulation of the invention may be used for treating seborrhea, also referred to as oily scalp.

Androgenic balding, telogen effluvium, as well as other conditions that cause hair loss, may cause scalp sensitivity. Such scalp sensitivity may include symptoms such as burning, itching, numbness, and stinging.

The formulation of the invention was shown to reduce skin tenderness and sensitivity characterized by itching.

Accordingly, the formulation of the invention may be used for treating skin sensitivity associated with hair loss.

The formulation of the invention was also shown to improve the color of the scalp, especially in elderly subjects. After treatment with the formulation of the invention, the pale color of the scalp in elderly subjects resumed a color and brightness typical to a chronologically younger scalp. The increase in the vitality of the skin was highly pronounced in elderly subjects.

The formulation of the invention is well tolerated and does not cause any systemic or local side effects, specifically it does not cause local irritation, and does not cause erectile disfunction or reduction in sexual desire.

The formulation comprises several components each contributing a different functional effect culminating in the total efficacy of the formulation. The invention therefore provides a formulation comprising components which extend the anagen phase of the hair, provide sulfates, effective inhibition of the enzyme 5-alpha-reductase, antiinflammatory effects, and reduction in the oxidative damage to the skin and hair follicles.

The present invention therefore provides a hair care formulation comprising:
(**a**) base oil;
(**b**) Arnica montana extract;
(**c**) green tea extract;
(**d**) Salvia rosmarinus extract; and
(**e**) onion oil.

Preferably, the base oil is almond oil.

The Arnica montana extract is present in an amount of between about 45% and about 55% volume/volume (v/v) of the formulation. Specifically, in an amount of about 50% v/v.

The green tea extract is present in an amount of between about 10% and about 20% v/v of the formulation. Specifically, in an amount of about 15% v/v.

The Salvia rosmarinus extract is present in an amount of between about 10% and about 20% v/v of the formulation. Specifically, in an amount of about 15% v/v.

The onion oil is present in an amount of between about 2.5% and about 7.5% v/v of the formulation. Specifically, in an amount of about 5% v/v.

The formulation of the invention is suitable for topical administration. Accordingly, in an embodiment, the present invention provides a topical hair care formulation.

In an embodiment, the topical hair care formulation of the invention comprises:
(**a**) almond oil;
(**b**) 50% v/v Arnica montana extract;
(**c**) 15% v/v green tea extract;
(**d**) 15% v/v Salvia rosmarinus extract; and
(**e**) 5% v/v onion oil.

In an embodiment, the formulation is locally administered by direct application onto the scalp, e.g., by applying drops (for example, between about 20 and about 30 drops, e.g., 25 drops) onto the scalp from the first hair line to the crown, followed by thorough massaging of the formulation into the skin of the scalp.

The formulation may be kept on the scalp for varying periods of time between about 40 minutes and several hours (e.g., overnight). If the application is performed for a short period of time (e.g., 40 minutes), the scalp may be covered by a sealing coverage (e.g., a bath cap), and slightly warmed for example using a hair dryer or a warm towel.

After treatment, the formulation is removed by washing the hair.

In one embodiment, the formulation is administered once every two weeks, once a week, twice a week, three times a week, four times a week, five times a week, six times a week, or daily. In one embodiment, the formulation is administered three times a week, and after an improvement becomes evident the formulation may be administered once a week or once every two weeks for an unlimited time, for example for one year, two years, three years or more, or throughout the subject's lifespan.

The formulation may be administered once symptoms of hair loss become evident.

In another embodiment, the formulation is administered as a prophylactic treatment in subjects that are prone to suffer from hair loss.

The formulation of the invention may be administered as an oil by dripping directly onto the scalp, it may also be incorporated into a hair mask, conditioner and/or shampoo.

As used herein the term ***"human subject"*** refers to both men and women, namely to a male human subject and to a female human subject. The formulation of the invention may be applied to subjects at any age, for example to men and women at their 20's, 30's, 40's, 50's and older.

In another aspect, the present invention provides a method of preventing, attenuating, or treating hair loss in a human subject comprising topical administration of the formulation of the invention.

The effect of the formulation is apparent from the first use. Without wishing to be bound by theory, the immediate effect appears to result from the ability of the formulation to extend the anagen phase of the hair and thereby contribute to reduction in hair shedding from the first uses.

The term ***"about"*** as used herein indicates values that may deviate up to 1%, more specifically 5%, more specifically 10%, more specifically 15%, and in some cases up to 20% higher or lower than the value referred to, the deviation range including integer values, and, if applicable, non-integer values as well, constituting a continuous range.

It must be noted that, as used in this specification and the appended claims, the singular forms "a", "an" and "the" include plural referents unless the content clearly dictates otherwise.

Throughout this specification and the Examples and claims which follow, unless the context requires otherwise, the word ***"comprise",*** and variations such as ***"comprises"*** and ***"comprising",*** will be understood to imply the inclusion of a stated integer or step or group of integers or steps but not the exclusion of any other integer or step or group of integers or steps.

Disclosed and described, it is to be understood that this invention is not limited to the specific examples, methods' steps, and compositions disclosed herein as such methods' steps and compositions may vary somewhat. It is also to be understood that the terminology applied herein is used for the purpose of describing specific embodiments only and not intended to be limiting since the scope of the present invention will be limited only by the appended claims and equivalents thereof.

### EXAMPLES

### Application of the formulation of the invention improves the hair condition in subjects suffering from MPB

### Composition and mode of application:

A formulation comprising:
(**a**) almond oil;
(**b**) 50% v/v Arnica montana extract;
(**c**) 15% v/v green tea extract;
(**d**) 15% v/v Salvia rosmarinus extract; and
(**e**) 5% v/v onion oil.
was locally administered by direct application onto the scalp of about 25 drops of the formulation spread from the first hair line to the crown, followed by thorough massaging of the formulation into the skin of the scalp.

The formulation was kept on the scalp overnight and was removed by washing the hair.

The formulation was administered three times a week for at least six months.

### Results:

A man aged 30, suffering from Male Pattern baldness (MPB), also referred to as androgenic baldness, received treatment as indicated above. As can be seen in Figure 1, six months after commencement of treatment, a significant improvement was observed in the amount and density of the hair. In addition, a significant reduction was observed in the oiliness of the scalp.

A woman aged 53, suffering from MPB, received treatment as indicated above. As can be seen in Figure 2, already 3.5 months after commencement of treatment, and even more pronounced at eight months after treatment, a significant improvement was observed in the amount, quality, and density of the hair.

A man aged 29, suffering from MPB, received treatment as indicated above. As can be seen in Figure 3, six months after commencement of treatment, a significant improvement was observed in the amount and density of the hair. In addition, a significant reduction was observed in the oiliness of the scalp, in the amount of dandruff, and in the skin tenderness and sensitivity that was characterized by a high degree of itching prior to commencement of the treatment.

A man aged 38, suffering from MPB, received treatment as indicated above. As can be seen in Figure 4, six months after commencement of treatment, a significant improvement was observed in the amount and density of the hair. In addition, a significant reduction was observed in the oiliness of the scalp.

A man aged 22, suffering from MPB, received treatment as indicated above. As can be seen in Figure 5, six months after commencement of treatment, a significant improvement was observed in the amount and density of the hair. In addition, a significant reduction was observed in the oiliness of the scalp.

A woman aged 37, suffering from MPB, received treatment as indicated above. As can be seen in Figure 6, seven months after commencement of treatment, a significant improvement was observed in the amount, quality, and density of the hair. In addition, a significant reduction was observed in the oiliness of the scalp.

A woman aged 47, suffering from MPB, received treatment as indicated above. As can be seen in Figure 7, already 2.5 months after commencement of treatment, and even more pronounced at six months after treatment, a significant improvement was observed in the amount and density of the hair. In addition, a significant reduction was observed in sensitivity of the scalp to hair coloring procedures.

## Claims

1. A hair care formulation comprising:
(**a**) base oil;
(**b**) Arnica montana extract;
(**c**) green tea extract;
(**d**) Salvia rosmarinus extract; and
(**e**) onion oil.

2. The hair care formulation of claim 1, wherein said base oil comprises almond oil.

3. The hair care formulation of claim 1 or claim 2, wherein said Arnica montana extract is present in an amount of between about 45% and about 55%, or at about 50%, volume/volume (v/v) of the formulation.

4. The hair care formulation of any one of the preceding claims, wherein said green tea extract is present in an amount of between about 10% and about 20%, or about 15%, volume/volume (v/v) of the formulation.

5. The hair care formulation of any one of the preceding claims, wherein said Salvia rosmarinus extract is present in an amount of between about 10% and about 20%, or at about 15%, volume/volume (v/v) of the formulation.

6. The hair care formulation of any one of the preceding claims, wherein said onion oil is present in an amount of between about 2.5% and about 7.5%, or at about 5%, volume/volume (v/v) of the formulation.

7. The hair care formulation of any one of the preceding claims wherein said formulation is administered topically.

8. The hair care formulation of any one of the preceding claims comprising:
(**a**) almond oil;
(**b**) 50% v/v Arnica montana extract;
(**c**) 15% v/v green tea extract;
(**d**) 15% v/v Salvia rosmarinus extract; and
(**e**) 5% v/v onion oil.

9. The hair care formulation of any one of the preceding claims for use in preventing, attenuating, or treating hair loss in a human subject.

10. The hair care formulation of claim 9 wherein said human subject suffers from a condition selected from a group consisting of androgenic balding, telogen effluvium, medication-induced hair loss, scalp inflammation, and ichthyosis vulgaris.

11. The hair care formulation of any one of claims 1 to 8 for use in improving hair health and appearance in a human subject.

12. The hair care formulation of any one of claims 1 to 8 for use in reducing scalp oiliness and/or sensitivity.

13. A kit comprising:
(**a**) the hair care formulation of any one of claims 1 to 8; and
(**b**) instructions for use in preventing, attenuating, or treating hair loss, for use in improving hair health and appearance, or for use in reducing scalp oiliness and/or sensitivity.

14. The kit of claim 13, wherein said instructions comprise massaging the formulation onto the scalp and keeping the formulation on the scalp for between about 40 minutes and several hours (e.g., overnight), prior to removal by washing the hair.
